# EUROPEAN PATENT SPECIFICATION

(11) **EP 0 374 243 B1**
(45) Date of publication and mention of the grant of the patent: **10.04.1996**
(21) Application number: 89907538.6
(22) Date of filing: 07.06.1989
(51) Int. Cl.: A61B 17/36

(54) **MEDICAL DEVICE APPLYING LOCALIZED HIGH INTENSITY LIGHT AND HEAT, PARTICULARLY FOR DESTRUCTION OF THE ENDOMETRIUM**
MEDIZINISCHE ANORDNUNG ZUR ANWENDUNG VON LOKALISIERTEM LICHT UND LOKALISIERTER WÄRME HOHER INTENSITÄT, INSBESONDERE ZUR VERNICHTUNG DES ENDOMETRIUMS
DISPOSITIF MEDICAL APPLIQUANT DE LA LUMIERE ET DE LA CHALEUR LOCALISEES A HAUTE DENSITE, NOTAMMENT POUR LA DESTRUCTION DE L'ENDOMETRE

(30) Priority: 10.06.1988 US 205218
(43) Date of publication of application: 27.06.1990
(73) Proprietor: TRIMEDYNE LASER SYSTEMS, INC., Tustin California 92680 (US); EVERETT, Royice B., Edmond, OK 73034 (US)
(72) Inventor: EVERETT, Royice, B., Edmond, OK 73034 (US); ACOSTA, George, M., Long Beach, CA 90807 (US); HUSSEIN, Hany, M., G., Costa Mesa, CA 92626 (US)
(74) Representative: Heath, Derek James
(86) International application number: US8902492
(87) International publication number: WO8911834

(56) References cited:
- EP-A- 0 182 689
- US-A- 4 445 892
- US-A- 4 662 368
- US-A- 4 672 961
- US-A- 4 736 743
- US-A- 4 740 047
- US-A- 4 773 413
- US-A- 4 781 681

## Description

### Technical Field Of The Invention

The present invention relates to medical devices for applying localized heat to a site in a patient's body, particularly for purposes such as the excising of tissue or deposits, or the cauterizing or destruction of tissue.

### Background Of The Invention

Localized heat applied to a site in a patient's body has often been used to cauterize a lesion in order to stop bleeding. Localized heat can also be used to alter, remove, or destroy tissue in a patient's body. One example of the medical use of localized heating is in the treatment of a bleeding ulcer. An endoscope is inserted through a patient's esophagus to view the bleeding site and to guide an electric powered heating element to contact the site and cauterize the bleeding. Another example is the use of localized heating to remove neoplastic pulmonary tissue. Still another example is the use of such heating to cauterize the endometrium.

Unfortunately, electric heating elements can be both difficult to manipulate and slow heating. The heating rate and maximum sustainable temperature are limited by the electric current available to the element. The available current in turn is limited by the size of the wires leading to the element. Wire size limits access to body sites for two reasons: larger wires cannot be inserted into small areas, and increased wire size typically causes a loss of flexibility.

The electric current passing through the wires also limits the regions in the body in which such a device can be used. The current presents a threat of an electric shock to the patient. The electric field generated by flowing current can also have undesirable effects. One region where such an electric field could possibly be life threatening is in the heart.

One electrically heated medical device in which the end of an endoscope is heated to avoid dew forming on a window is shown in US-A-4,279,246. That device heats the window to about body temperature to prevent dew formation. However, due to the design of the device, the heat generated on the window is limited to about body temperature and therefore cannot be used to alter or destroy tissue.

Another electrically heated medical device that becomes sufficiently hot so as to cauterize tissue is shown in US-A-4,449,528. A miniaturized, endoscopically deliverable thermal cautery probe is used to cauterize internal vessels. The probe is applied to tissues cold, and a large number of electric heating pulses of equal energy are then applied to an internal heating element within the probe. The probe's internal heating element is in direct thermal contact with an active heat-transfer portion that has a low heat capacity. The low heat capacity of the heat-transfer portion insures quick heating and subsequent cooling, thereby adequately coagulating tissue while minimizing heat penetration and resulting tissue damage.

Because of the difficulties with electrical heating, medical devices, systems and methods have been developed for applying localized heat that is generated otherwise than by routing an electric current to a site in a patient's body. The localized heat so generated can be used for several purposes. For example, it may be used to cauterize a lesion to stop bleeding, to remove a clot, or to remove an arteriosclerotic deposit from a blood vessel. The localized heat can also be used to create an open channel in a previously occluded blood vessel.

One medical device not employing electrical current for heating is described in US-A-4,207,874 which discloses a laser tunneling device used to locate, analyze, illuminate and destroy obstructions in a lumen such as a blood vessel. The device includes a fiberoptics bundle in a flexible conduit that is insertable into the blood vessel. The conduit includes a connection to a suction source at one of its ends, a valved means of controlling the application of suction which also functions to control the injection of locating material, and a connection to the fiberoptics bundle. The fiberoptics bundle is divided into an illuminating source bundle portion, a viewing bundle portion and a laser bundle portion. The device functions to remove obstructions in tube structures of both biological and non-biological types by insertion of the conduit sheathed device into the tube structure in a position distal to the obstruction.

A still further prior medical device contemplates use of a single fiberoptic light transmission path within a medical catheter device to be either a viewing system, a laser light transmitting system, or a combination of both. In US-A-4,445,892 a dual balloon catheter device is shown to have two spaced and expandable balloons for occluding a segment of a blood vessel. An optic system is used in the segment for viewing or for delivering laser light. Both the viewing and the laser light delivery are through a circumferential window within the tubular structure of the catheter.

US-A-4,646,737 describes a device that includes a heat-generating element mounted on the distal end of an elongated electromagnetic energy transmitting conduit or member. A preferred conduit is a single flexible quartz optical fiber. Electromagnetic energy in the form of visible light from an intense light source, such as a laser, is transmitted through the conduit and is emitted onto a light-receiving surface of the heat-generating element. The light is converted by the element to heat. The heated element is then placed in contact with material in a patient's body such as a clot, deposit or tissue. The heated elements alter the material by melting, removing or destroying it. The heat-generating element preferably has a rounded exterior surface end. It is typically retained on the conduit by a locking means, such as by a ridge on the element that is received in a complementary groove on the conduit.

Still other prior medical devices tunnel and cut bodily tissue and other material within the body by direct application high intensity, typically laser, light that is typically conducted through fiberoptics. Laser devices --the acronym "laser" indicating light amplification by the stimulated emission of radiation -- are well known. Briefly, a laser device operates by using an intense source to cause ions to become inverted with respect to their normal energy distribution. The tendency of such ions is to relax to a so-called "ground state" (a normal distribution), and in so doing to stimulate inversion of other ions within the same wavelength. A synchronized output is promptly achieved wherein the ion's relaxations from an inverted energy state transpire in unison. A massive output of energy is thereby obtained. The output wavelength is determined by the difference between the energy level from which the ions relax and the ground state energy level which the relaxed ions assume.

The medical device shown in US-A-3,315,680 describes a cauterizer using fiberoptic techniques to conduct ordinary and laser light in a medical application. US-A-3,821,510 shows the use of a laser system which accommodates fluid flow to control the temperature of the work area.

DE-A-2,826,383 shows a tubular probe for laser surgery that is placed against or inserted in tissue. In one embodiment, an end piece having an absorbent surface is heated by the beam while it is in contact with the tissue, thereby heating the tissue. Alternatively, in another embodiment, the end is transparent and permits the laser beam to pass through the end in order to radiatively heat the tissue.

These various types of prior medical devices do not permit that tissue destruction using the lateral direction of high intensity radiated light and using radiated and/or conducted heat should be performed closely proximately, or simultaneously, in time. This can be very useful when it is desired to destroy large surface areas of tissue to a substantial depth.

For example, a surgical procedure referred to as "endometrial ablation" has been recently developed as an alternative to hysterectomy for treatment of excessive uterine bleeding. In this procedure, an Nd:YAG laser is used to destroy the entire endometrium lining the uterus. An optical fiber is inserted in the uterus by means of a hysteroscope to conduct the laser energy to the endometrium. With the aid of a parallel optical viewing fiber of the hysteroscope, the end of the laser-transmitting fiber is slowly moved across the surface of the endometrium so that the laser energy penetrates and destroys the endometrium which is on the order of three millimeters thick. Typical prior art procedures have utilized a bare optical fiber for transmitting the laser energy. Two techniques have been developed. By one technique, the end of the bare optic fiber is actually touched to the endometrium. By a second technique, generally referred to as "blanching", the bare tip of the optic fiber is held several millimeters away from the endometrium. These techniques are generally described in Daniell et al., "Photodynamic Ablation of the Endometrium With the Nd:YAG Laser Hysteroscopically as a Treatment of Menorrhagia", Colposcopy & Gynecologic Laser Surgery, Vol. 2, No. 1, 1986; Mackety, "Alternative to Hysterectomy: Endometrial Albation by Laser Photovaporization", Today's OR Nurse, Vol. 8, No. 4; and Goldrath et al., "Laser photovaporization of endometrium for the treatment of menorrhagia", Am. J. Obstet. Gynecol., Vol. 140, No. 1, page 14, May 1, 1981.

Some surgeons prefer the "blanching" technique because it is believed to create fewer complications. There is less danger of mechanical perforation of the uterus. There is less actual vaporization and cutting of the endometrial tissue and accordingly less fluid absorption thereby.

It is difficult, however, to treat the side walls of the uterus by "blanching" due to lack of room to maneuver the optic fiber so as to direct it toward the side walls. Thus a touching or dragging technique has necessarily been utilized during those portions of the procedure. In addition to being unable to direct the laser energy directly at the side wall of the endometrium, this touching of the fiber tip to the endometrium is, as mentioned, considered undesirable by some surgeons.

Furthermore, with the touching technique, and to a lesser extent with the blanching technique, there is always the problem of completely treating the entire endometrium without missing small areas here and there.

Accordingly, it would be desirable if a medical device were available which would permit more of the laser energy to be directed transversely from the optical fiber toward the side wall of the endometrium. It would further be desirable to maintain some suitable spacing between the tip of the optic fiber and the endometrium. Also, it is desirable that heat be conductively applied to the endometrium while simultaneously directing the laser energy to a more localized spot thus better insuring destruction of the entire surface of the endometrium.

It would additionally be useful if the operative excising and cauterizing head of the device were to somehow be directional, as well as necessarily controllable, in one or both of its light radiation and/or its conductive heating effects. A preferred operational direction of the device, operative head for either the lateral transmission of light or the conductive heating of tissue would permit that one effect could be maximized over the other by action of the surgeon's positioning and orientation of the device's operative head within the body cavity. Furthermore, a device exhibiting a preferred directionality would presumably exhibit some safe orientation in which orientation the device's operative head would not be prone to destroy and/or burn the lining of the body cavity within which it was situated.

### Summary of the Invention

With the above considerations in mind, the present invention is directed to a medical device having the construction specified in claim 1, the preamble of that claim being based on what is shown in US-A-4 662 368.

An essential difference between the present invention according to the characterizing portion of claim 1 and what is shown in the above-specified U.S. Patent is that the present invention utilises a beam splitter means which directs laser radiation transversely to the axis of the laser conduit through a laterally placed aperture of a distal cavity of the device.

Preferred features of the present invention are set forth in the subsidiary claims.

The laser energy used by the medical device in accordance with the present invention may be infrared (IR) and excimer laser light as well as visible laser light. Various suitable energies are produced by CO₂, Argon, Nd:YAG, excimer and other types of lasers. Particularly when the beam-splitting means of the device is implemented as a reflective surface of gold (Au), the device is very effective in providing both conductive and heat energy for a range of laser radiation frequencies, types, and energies.

Numerous other advantages and features of the present invention will be readily apparent to those skilled in the art from the following detailed description of the preferred embodiment of the invention, the drawings, and the appended claims.

### Brief Description Of The Drawings

FIG. 1 is a schematic view of a system including a medical device embodying the present invention in use within a human uterus.
FIG. 2 is an enlarged side view, partly in section, of the distal end portion of the medical device of FIG. 1.
FIG. 3 is an enlarged end view of the distal end portion of the medical device of FIG. 1.
FIG. 4 is an enlarged cross-sectional view of the distal end portion of the medical device, taken along PLANE 4-4 in FIG. 2, with a portion broken away to show additional detail.
FIG. 5 is an enlarged cross-sectional detail view of the distal end portion of the medical device of FIG. 1; and
FIGURES 6-12 are similar to FIGURE 5 and illustrate alternative embodiments of the present invention.

### Description Of The Preferred Embodiment

While the present invention can be embodied in many different forms, there is shown in the drawings, and described in detail, a preferred embodiment of the invention. The present disclosure is an exemplification of the principles of the invention and is not intended to limit the invention to the embodiment illustrated.

The present invention is a medical device for delivering and applying localized heat as well as a laser beam to a site upon, or more typically in, a patient's body. The applied energy can be used to selectively stop bleeding, or to remove or alter a material such as tissue or deposit in or on the body by vaporization, or to make an incision. The material being altered can be any solid or semi-solid substance found in or on the body including living tissue (including cancerous tissue) or deposits such as clots, fat or arteriosclerotic plaque. A particularly useful application of the invention is the destruction of the endometrium lining the uterus.

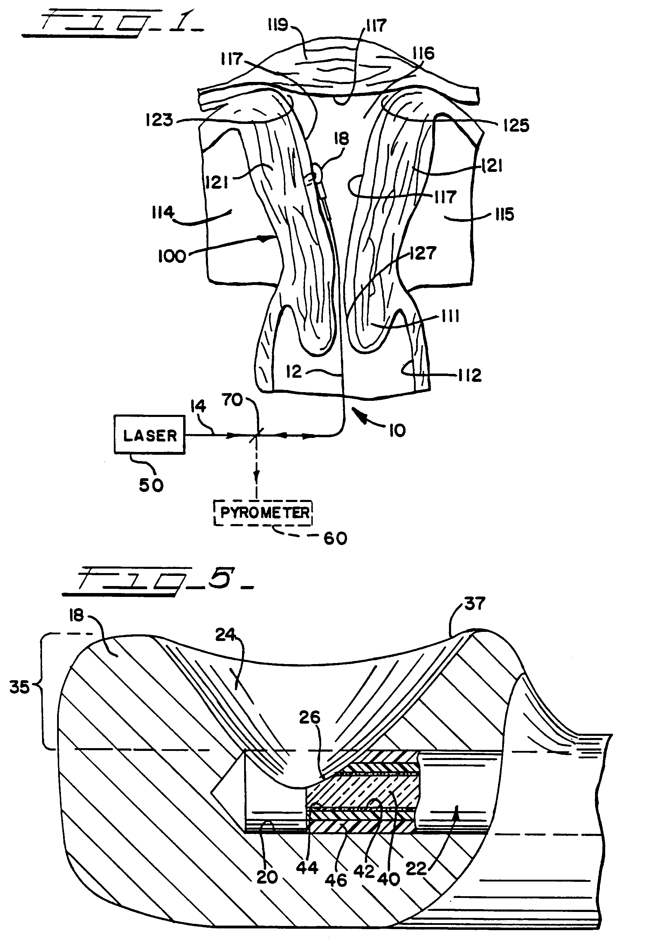

Referring to FIG. 1, medical device 10 embodying the present invention is shown positioned within the uterus 100 of a human female patient. Device 10 comprises hollow, apertured bulbous element 18 positioned within uterine cavity 116 near endometrium 117. The endometrium 117 is a thin layer of tissue lining the entire uterine cavity 116 which is defined by an upper fundus 119 and a somewhat cylindrical side wall 121. The side wall 121 can generally be defined as extending from the tubal ostia 123 and 125 down to the internal cervical os 127.

Bulbous element 18 is mounted on the distal end of elongated, laser energy transmitting conduit such as fiber optic 22 which, in turn, is optically coupled at its proximal end 14 to laser energy source 50. Optionally, a pyrometer 60 is provided at the proximal end region of device 10 for measuring the temperature of bulbous element 18. To that end, a reflected beam from bulbous element 18 is conducted to pyrometer 60 utilizing a second beam-splitting means 70 such as a partial mirror, a rotating mirror, or the like.

Device 10 is inserted as part of a hysteroscope (not shown) into uterus 100 via vaginal canal 112 and through the internal cervical os 127 of cervix uteri 111 using appropriate dilation procedures as will be described in greater detail hereinbelow. The body or side wall 121 of uterus 100 is supported by broad ligaments 114 and 115.

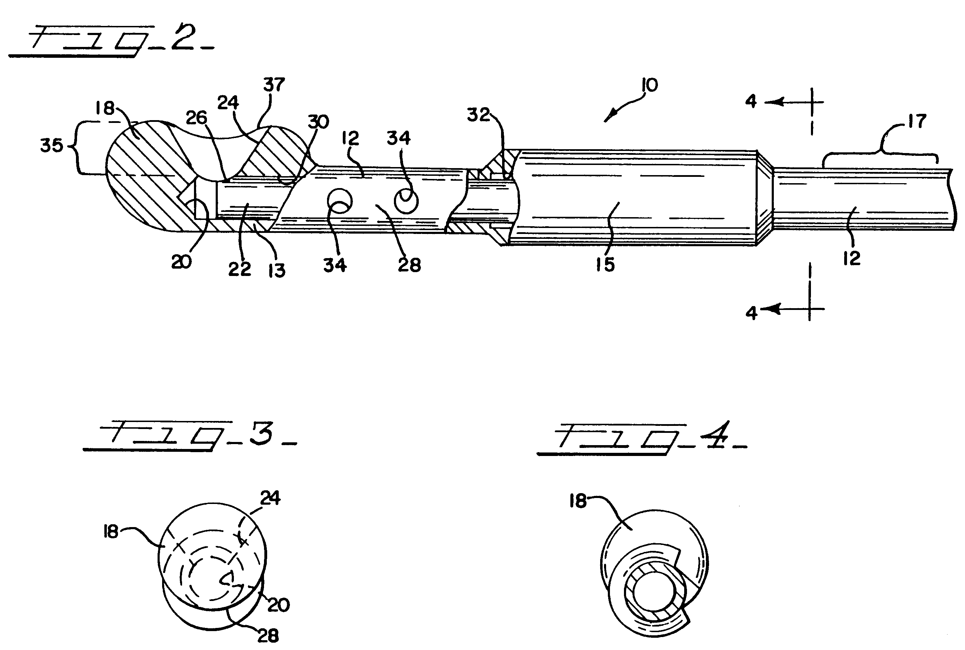

An enlarged view of the distal end portion of device 10 is shown in FIG. 2. Bulbous element 18 defines an internal cavity 20 within which is slidably received the distal end of fiber optic 22 which serves as the laser energy transmitting conduit. A clearance is provided between the distal end of fiber optic 22 and cavity 20 to accommodate differences in thermal expansion that may be encountered upon the heating of bulbous element 18. Bulbous element 18 also defines aperture 24 that communicates with cavity 20 and provides an exit passageway for a laser energy beam diverted from the laser energy path of the laser energy entering cavity 20 via fiber optic 22. Diversion of the laser energy beam is achieved by beam-splitter means 26 which in one embodiment is a concave side portion of fiber optic 22 with laser light reflecting cladding removed therefrom. The beam splitter means 26 can also be a rounded end of fiber optic 22 without a reflective cladding, or a separate lens, e.g., a sapphire lens, positioned at the terminus of fiber optic 22, that reshapes and redirects the laser beam.

The beam splitter means may still further be a reflective surface, or mirror, on the wall of cavity opposite to the distal end of fiber optic. The reflective surface may be positioned so as to intercept all or part of the laser energy beam emitted from the distal end of fiber optic, and may be of varying reflective efficiency so as to reflect substantially all or only part of the intercepted laser energy beam. The reflective surface may be varied in its position, size, and/or efficiency to adjust the relative level of energy converted into heat versus that which exits from the bulbous element 18 as a laser energy beam.

The operation of various embodiments of the beam-splitter means will be described in greater detail hereinbelow.

The internal cavity 20 of bulbous element 18 exhibits features 32-34 that facilitate the reception and clenched retention of bulbous element 18 upon fiber optic 22. The fiber optic 22 generally has a total exterior diameter or transverse dimension of about one (1) millimeter or less. Nonetheless, it generally has sufficient rigidity both to be pushed into a narrow, complementary sized, bore region 30 of cavity 20. The fib er optic 22 is facilitated in being guided into a tight fit within narrow bore region 30 by guidance accorded the fiber optic 22 in large bore region 32 to cavity 20.

When inserted to the indicated depth within bore regions 30, 32 of cavity 20, and properly rotated so that beam-splitter means 26 is properly aligned to aperture 24, the fiber optic 22 may be inspected through holes 34 defined in the sidewall of stem 12 connected to bulbous element 18. Holes 34 also provide a vent means that serves to relieve any pressure that may be generated within the bulbous element 18 due to gas expansion or vaporization of liquid as element 18 is heated. Additionally, holes 34 reduce the cross-sectional area available for heat transmission along the outer sheath 13 of hollow stem 12 unitary with bulbous element 18. Outer sheath 13 is further provided with a thermal expander section 15 which is an enlarged cylindrical segment unitary with stem 12 that serves to accommodate thermal expansion of stem 12 as bulbous element 18 is heated.

Fiber optic 22 is secured to stem 12 in the region 17 immediately behind or abaft of thermal expander section 15, preferably by crimping or like mechanical securement means. Alternatively, or in addition, a heat resistant adhesive such as an epoxy glue may be used.

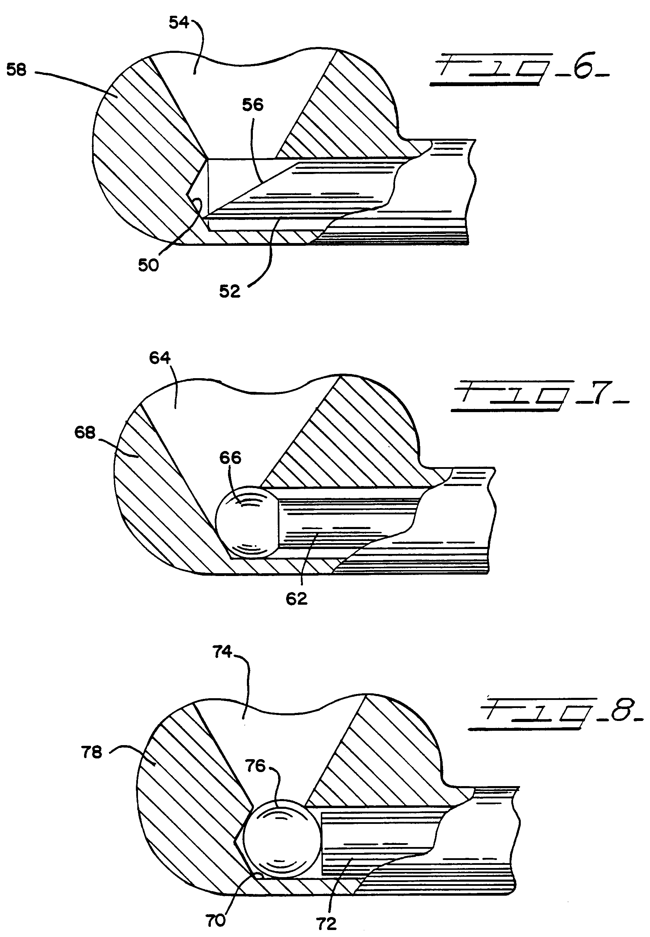

An enlarged distal end view of the medical device 10 is shown in FIG. 3. The aperture 24 shown in FIGS. 2 and 3 is preferably of a sophisticated contour. It subtends an arcuate portion of the circumference of bulbous element 18, and of the circumference of fiber optic 22. This arcuate portion is typically less than one quadrant of 90 . The overall aperture 24 is substantially in the shape of a frustum. The wide base of the frustum is disposed to the exterior of the bulbous element 18. The truncated apex of the frustum is abutting the distal end region of the light-transmitting fiber optic 22 at the position of its beam-splitter means 26. Because the cross section of aperture 24 is substantially circular, it may be accurately described as being substantially frustoconical in shape.

There is a spacing or standoff 35 defined between fiber optic 22 and an outer surface 37 of bulbous element 18 which will be contacted with the endometrium. This distance 35 is on the order of the diameter of fiber optic 22, i.e., 1 millimeter.

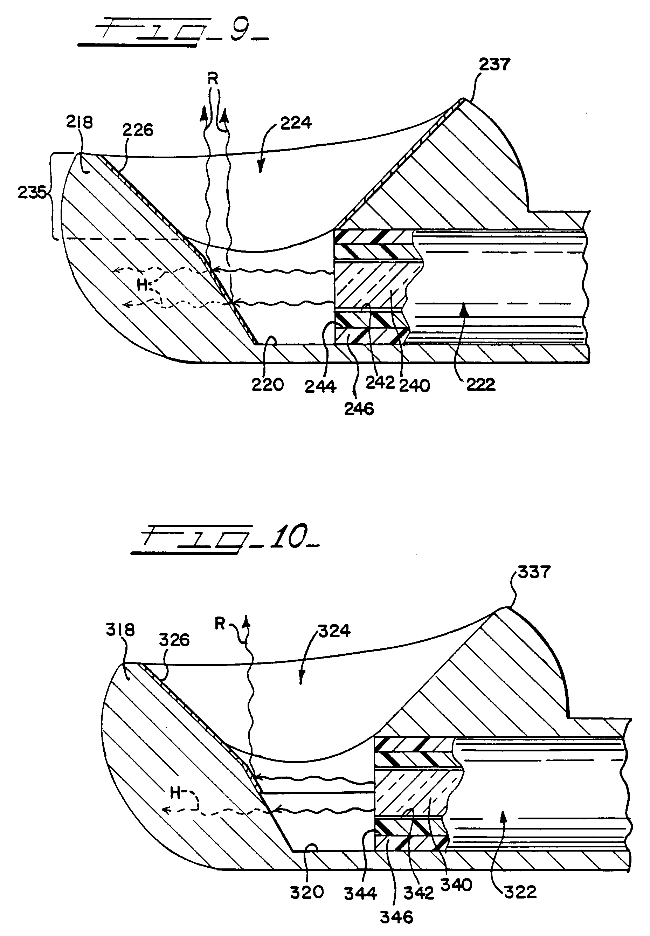

An enlarged cross-sectional view of the bulbous element taken along plane 4-4 shown in FIG. 2 is shown in FIG. 4. The axial offset between the distal head and the proximal shank regions of bulbous element 18 facilitates maneuvering of the prominent distal head region of the element 18 into proximity or contact with tissue while, at a preferred angular orientation of use, the distal shank region of the element 18 remains more remote from the tissue. Tissue selective contact with only proximal or distal regions of bulbous element 18 cannot be totally assured when the device 10 is used in tight bodily cavities such as the uterus 100 (shown in FIG. 1), but the axial offset to bulbous element 18 promotes that its distal head region may be selectively placed in contact with the tissue.

Further, the spacing 35 maintained between the distal end of fiber optic 22 and the tissue being treated minimizes the disadvantages mentioned that often result from actual touching of the fiber optic 22 to the endometrium.

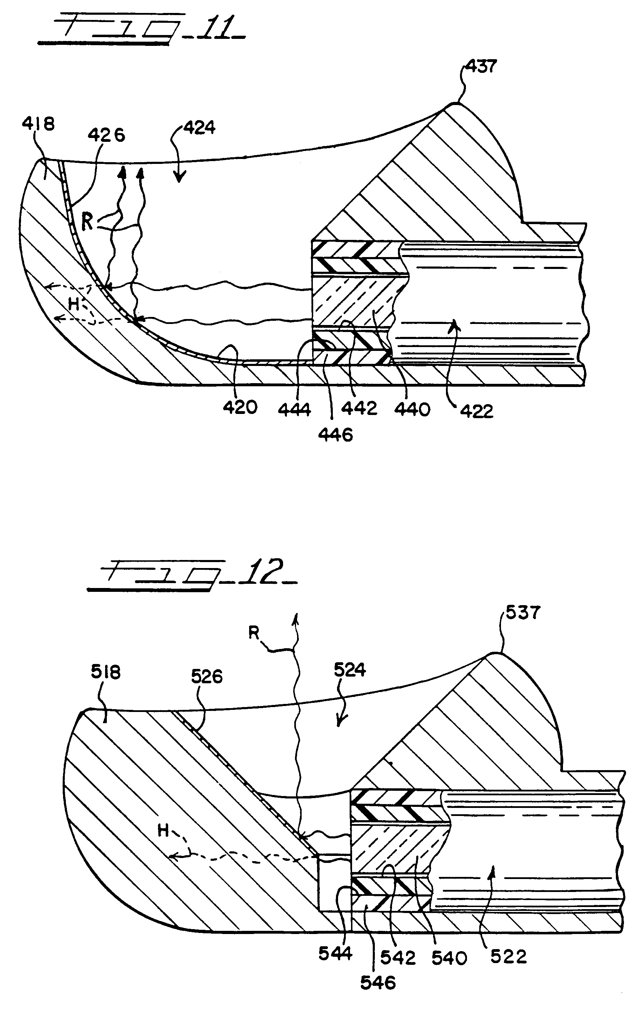

An enlarged cross-sectional view of the distal head region of bulbous element 18 is shown in FIG. 5. The fiber optic 22 includes a core 40 surrounded by cladding 42. The internal reflection caused by the cladding 42 is such that the fiber optic 22 has a low divergence as the light exits the distal end 16. The core 40 is typically made of glass, e.g., silica quartz. The cladding 42 is typically made of silicone, plastic or silica. The core 40 and its cladding 42 have a combined diameter of less than about 0.5 millimeter to about 1.0 millimeter.

To protect the core 40 and its cladding 42, the fiber optic 22 normally also includes an external jacket 46 which surrounds the cladding 42 and is held in place by a resin coating 44. The external jacket 46 is usually made of a flexible plastic material such as poly(ethylene) or poly(tetrafluoroethylene). It provides a flexible and smooth surface allowing easy manipulation of the medical device. Fiber optic bundles are not preferred since the adhesive between individual fibers limits the amount of light which can be transmitted without melting of the bundle.

The fiber optic 22 should be flexible yet sufficiently resilient so that it is possible to push the fiber optic along a lumen. One such suitable fiber optic having a core diameter of 0.4 millimeters is marketed under the designation Med 400 by Quartz Products Corporation of Plainfield, New Jersey. Another suitable fiber optic is a 0.6 millimeter fiber optic commercially available under the designation HCT 600 from Ensign Bickford Co., Connecticut. The power that can be transmitted along fiber optic 22 varies with the size of the fiber. Utilizing the HCT 600 fiber optic a medical device embodying this invention can transmit as much as about 60 watts continuous power from a Nd:YAG laser source.

The bulbous element 18 irradiates both heat and light energy. Part of the light energy transmitted by fiber optic 22 is partially absorbed and converted by the element 18 into heat, and part of the light energy is emitted by the element 18 through aperture 24 as light. The relative proportions of the light energy that is radiated as light, or that is radiated and/or conducted as heat, are determined by the beam-splitter means 26.

The cross-sectional area of core 40 to fiber optic 22 is divided by the beam-splitter 26 -- essentially a concave notch extending into the core 40 of the fiber optic 22 at the position of aperture 24 -- into a part that is perpendicular to the axis of fiber optic 22 and another part that forms an acute angle to such axis. Substantially all of the light exiting the perpendicular part of fiber optic 22 at its beam-splitter means 26, i.e., exiting axially from fiber optic 22, is directed forward to be absorbed by the opposed light-receiving surface of bulbous element 18. The light-receiving surface of bulbous element 18 that is opposed to the distal end of fiber optic 22 is preferably treated, e.g., oxidized, in order to increase its coefficient of emissivity to about 0.95 or greater. This treatment further increases the absorption of light by the element 18. Alternatively, the light-receiving surface can be treated by being coated by a material such as lamp or carbon black having a high coefficient of emissivity.

The bulbous element 18 is preferably made of metal such as surgical stainless steel, but could also be made of a combination of thermally conductive and thermally insulating materials such as metal(s) and ceramic(s). The exterior surface of the bulbous element 18 is preferably coated with a non-stick or release surface such as poly(tetrafluoroethylene) to provide easy release from the tissue. Poly(tetrafluoroethylene) usually is used for operating temperatures below about 300 degrees C. The majority of the heat is generated by absorption of the laser light at the distal end of the bulbous element 18 where it is typically needed. Meanwhile, heat generation is minimized at the proximal portions of the element 18 where it could be detrimental to the fiber optic 22.

The bulbous element 18 has sufficient mass to avoid burn-through during use. However, the mass is not so great as to materially slow its heating rate. For this reason, it is advantageous to place the thickest portion of material in the forward portion of the element 18 where the radiant energy, e.g., light, impinges. The space between the distal end of the fiber optic 22 and the radiant energy receiving surface of the element 18 may fill with matter such as air or liquid during use. However, this matter is readily vented through aperture 24 due to expansion as a result of the heat generated.

The distal end portion of the bulbous element 18 is preferably generally rounded on its exterior surface (as illustrated) in order to facilitate pressing the element into and through softened body material while minimizing the risk of mechanical perforation. The bulbous element 18 can alternatively have other shapes as desired, including oblong or eccentric with respect to the axis of the fiber optic 22 or even generally crescent shaped. Such an eccentric or oblong shape can be rotated to generate an even larger channel through an obstruction. A crescent-shaped element also allows for viewing past the element.

The distal end of the fiber optic 22 is preferably spaced no more than two diameters of its core 40 away from the light-receiving surface of the bulbous element 18. Where the core 40 is about 0.5 millimeters, this spacing should be no more than about 1 millimeter. This relatively close spacing insures that substantially all of the light emitted from the flat end surface of fiber optic 22 is received on the forward light-receiving surface of the bulbous element 18, and is not dispersed on the inside side walls of the cavity 20 between the distal end surface and the receiving surface.

Meanwhile, some light is diverted from optic fiber 22 by beam-splitter means 26 to be directly radiated from bulbous element 18. This radiation is in a direction substantially transverse to the axis of optic fiber 22. The beam-splitter means 26 is essentially an indentation or recess upon the fiber optic 22. The recess extends sufficiently deeply into the fiber core 40, as may be best observed in FIG. 5, so as to intercept a substantial portion of the light, and the light energy, which is transmitted along fiber optic 22 between laser light source 50 (shown in FIG. 1) and the fiber's distal end. The beam-splitter means 26 can be considered to create a lossy region, or region at which light is emitted, to the fiber optic 22.

The depth, and lineal extent, of the beam-splitter means 26 influences the total amount of the light energy that is radiated thereat. Typically, a variably predetermined portion of the light energy carried within fiber optic 22 may be radiated as light at the location of the beam-splitter means 26. The remaining light, and light energy, is transmitted to distal end of the optic fiber 22, radiated at that end, and absorbed by a light absorbent treatment or coating at the opposed surface of bulbous element 18. In this manner, the energy balance between localized heating performed by the element 18 due to emission of light versus local heating by thermally radiative and/or conductive paths may be predetermined in a controlled manner.

Alternative embodiments of the present invention, each having a different beam splitter means are illustrated in FIGS. 6-8. In particular, FIG. 6 shows a fiber optic 52 positioned within cavity 50 defined by bulbous element 58 and provided with a leveled or slanted end surface 56 that directs a portion of the transmitted laser energy outwardly through aperture 54 and at an acute angle to the major longitudinal axis of fiber optic 52.

Similarly, FIG. 7 shows fiber optic 62 terminating in a unitary spherical lens 66 that is positioned within bulbous element 68 and directs a portion of the transmitted laser energy outwardly via aperture 64.

FIG. 8 illustrates an embodiment where a separate spherical lens 76 is positioned at the very end of fiber optic 72 in cavity 70 of bulbous element 78 and directs a predetermined portion of the transmitted energy outwardly through aperture 74 while the remainder is absorbed by element 78 as heat.

FIGS. 9-11 illustrate embodiments where the beam splitter means is a partially reflective surface, or mirror, that is positioned and sized so as to reflect a portion of the energy emitted from the end of the fiber optic outwardly through the aperture while the remainder is absorbed as heat. Within FIGS. 9-11 structural elements performing a similar function to elements previously shown in FIG. 5 generally have the same last two digits in their reference numerals.

FIG. 9 illustrates an embodiment where the aperture 224 to bulbous element 228 is substantially in the shape of a trough having an included angle that is a right angle and major surfaces that intersect at the bottom of cavity 220. The aperture 224 can alternatively be configured in the shape of a wedge, cone, pyramid, paraboloid or other surface or body that is relatively wider at the exterior surface of bulbous element 218 and relatively narrower at its regions intersecting cavity 220 and fiber optic 240. At least the wall, or surface, of aperture 224 opposite to the end of fiber optic 240, and typically the entire surface of aperture 224, is reflective. The reflective surface 226 is preferably gold (Au), normally applied by plating. The preferred gold plating is that commercially available under the designation LASER GOLD (trademark of Epner Technology, Inc.) . This is an ultra-high infrared reflectance gold coating provided by Epner Technology Incorporated, 25 Division Place, Brooklyn, N.Y. 11222. The preferred gold plating is reported by its manufacturer to exhibit an absolute spectral reflectance of better than 40% at 0.5 microns wavelength radiation, and better than 98% from 1.0 to 12.0 microns wavelength radiation. The radio of laser energy radiation R reflected by surface 226 and directed outwardly from aperture 224 versus the laser energy heat H absorbed within the bulbous body 218 may accordingly be made high, which is sometimes especially desired when the radiation energy R is not focused. The reflective surface 226 may alternatively be made of silver (Ag), mercury (Hg), or other energy-reflective materials, and may alternatively be created by gaseous deposition and other processes as well as by the process of plating.

FIG. 10 illustrates an embodiment where the reflective surface 226 of aperture 324 to bulbous element 318 is not coextensive with the beam of laser radiation emitted from the end of fiber optic 340. Instead, the reflective surface intercepts only a portion of the laser energy radiation emitted from the end of fiber optic 340, and substantially reflects this intercepted portion through aperture 324 to the exterior of bulbous element 318. A remaining portion of the laser energy radiation emitted from the end of fiber optic 340 impinges upon a portion of the wall of cavity 324 that is substantially non-reflective, and is absorbed within the bulbous element 218 as laser energy generated heat H. The size, location, and reflectivities of each of the reflective and non-reflective surfaces of aperture 324 may be adjusted to vary the relative proportion of laser energy radiation that is reflected through aperture 324 and that is absorbed as heat within bulbous element 318. The extent of the reflective surface 326, in particular may be adjusted by selectively masked plating, or by removal of a portion of the reflective surface, however applied, by mechanical means such as drilling or grinding. In this context of the selective adjustment of the extent of reflective surface 326, it should be understood that FIG. 10 is exemplary only, and that the exact contours of aperture 324 and the exact patterning and location of the reflective surface 326 could be subject to considerable variation depending upon exactly where, how, and to what extent energy is to be both thermally and radially delivered by medical devices in accordance with the present invention.

As an example of the considerable adjustment that may be made to the contours of the aperture, and to a reflective surface within such aperture that is employed as the beam splitter means, FIG. 11 illustrates an aperture 424 to a bulbous element 418 where a partially reflective surface 426 within the aperture 424 directs some of the laser energy radiation received from fiber optic 440 in a direction outwardly from the tip of bulbous element 418, while permitting some of this laser energy radiation to be absorbed by the bulbous element 418 as heat. The direction of laser energy in an outwardly direction is provided by a reflective surface 426, normally made of gold. Reflective surface 426 is concavely curved, typically as a spheroidal, ellipsoidal, or parabolic surface. The reflective surface can but need not cover the entire aperture 424 defining wall. The concave curvature, and spatial orientation, of reflective surface 426 around aperture 424 not only causes that the laser energy is reflected to the exterior of the tip of bulbous body 418, but that it may be focused to a desired degree as well. Both the outwardly direction and the focus, or partial focus, of laser energy radiation R can be particularly useful when the medical device in accordance with the invention is moved in a progression to heat and irradiate material within, or regions of, a patient's body.

FIG. 12 illustrates still another embodiment of the beam splitter means. The aperture 524 to bulbous element 518, and its reflective surface 526 is not coextensive with the entire end of fiber optic 540. The reflective surface 526 is instead oppositely disposed to but a portion of the area of the end of fiber optic 540, and intercepts but a portion of the laser energy radiation emitted therefrom (similarly to the embodiment of FIG. 10). Only this intercepted portion of the laser energy radiation emitted from the end of fiber optic 540 impinges on reflective surface 526 and thus is substantially reflected through aperture 524 to the exterior of bulbous element 518. A remaining portion impinges directly onto the bulbous element 518 where it is converted into heat H.

The device in accordance with the present invention permits the destruction of tissue or matter, such as the endometrium. When the bulbous element 18 is positioned relatively further or relatively closer to the tissue surface then the light beam emitted from aperture 24 will fall upon the tissue surface relatively more or less diffusely. Since the light beam produced by the bulbous element 18 is not directed into the tissue surface as a narrow, collimated beam, any destruction by high-temperature localized heating with intense light will not continue to transpire as the bulbous member 18 is retracted ever further away from the work surface.

In accordance with the present invention, a medical device is used for radiant heating of material within a patient's body, including the patient's own tissue, by irradiation with high intensity light. The light is sufficiently localized and sufficiently intense so as to cause sufficiently localized sufficiently high radiant heating of the material upon which the light selectively impinges so as to destroy such material. In accordance with the preceding discussion, this destruction will be understood not to transpire uncontrollably in all regions whereat the aperture distal end of the device is deployed, but to transpire only selectively along an arc in a direction that is substantially transverse to the long axis of the distal end of the device.

Further in accordance with the present invention, a medical device is used for conductively heating tissue of the patient's body, typically in regions local to the region(s) of tissue or material destruction by the high intensity light. This conductive heating typically occurs by direct thermal contact with an apertured distal end of the device while this end is heated sufficiently hot so as to cause localized cauterizing of the patient's bodily tissue with which it comes into contact. The heated device generally does not, however, produce heat that is either so localized or so high as that heat that is produced by the high intensity light radiation also emitted transversely from the device's distal end.

When the device of the present invention is utilized in an endometrial ablation procedure as previously discussed, it is especially useful for treating the portions of the endometrium 117 lining the side wall 121 of the uterus. Although not shown in the drawings, the optic fiber 22 and bulbous element 18 are inserted into the uterus by means of a hysteroscope as will be understood by those skilled in the art. Use of the element 18 will be under direct visual observation through the hysteroscope. The bulbous element 18 will be placed in contact with the endometrium 117 with the aperture 24 directed against the wall of the endometrium 117 as indicated in FIG. 1. The bulbous element 18 will then be slowly moved in a continuous motion so as to direct the heat energy therefrom across the entire portion of the endometrium lining the side wall 121.

The endometrium, when suitably prepared for this procedure, will have a thickness of approximately three millimeters. The laser energy exiting the aperture 24 and directed immediately against the endometrium has the ability to penetrate approximately five millimeters, thus penetrating the entire thickness of the endometrium through to the underlying muscle layers. Additionally, the somewhat greater area of less intense heating provided by conductive heating from the bulbous element 18 due to its contact with the endometrium 117 surrounds the localized area of heating provided through the aperture 24. It is the combined heating effect of both the laser energy exiting aperture 24 and the heat conducted from the heated mass of bulbous element 18 which destroys the endometrium.

Typically, the tissue of the endometrium is not vaporized, but instead is heated to an extent that it is completely penetrated by the high temperatures and is thus entirely destroyed or killed. After treatment, the endometrial tissue typically is reduced to a scar tissue on the myometrial or muscle layer of the uterine cavity.

Also, it is noted that on occasion an actively bleeding vessel will be encountered in this procedure. In such instances, it is sometimes preferable to rotate bulbous element 18 and contact the bleeding vessel with a back surface thereof to cauterize the vessel without applying direct laser energy.

The terms "laser energy" and "laser radiation" and "laser light" as used in this specification disclosure will be understood to encompass a broad range of radiation frequencies, characteristics, and energy densities. In particular, the device in accordance with the present invention will function satisfactorily with laser radiation of a broad frequency range of infrared (IR) and visible light. The laser radiation may be suitably produced by CO₂, Argon, Nd:YAG, and other types of lasers. Use of a beam splitter having a reflective surface plated with gold, as is preferred, is effective to accomplish the heating and radiating purposes of the invention over a great range of energy frequencies, characteristics, densities, and levels. For example, the device in accordance with the present invention is suitable for use with excimer laser radiation having a wavelength in the order 290-400 nm and power densities, pulse rates, and application times sufficient to cause multiphoton absorption and band breaking by coulomb repulsion rather than thermal destruction.

In accordance with the preceding discussion, further adaptations and variations of the present invention will be readily perceived by a practitioner of the medical instrumentation arts. The size, aspect ratio, and contours of the bulbous element 18 can be adjusted as besuit the bodily cavity within which such member is employed. The focus of the emitted light can be varied in a sophisticated manner by incorporation of one or more lenses of standard design into aperture 24. Heat conduction within the bulbous element 18 may be varied by making the element out of both thermally conductive and thermally insulative material. The operation of that embodiment of the invention which has been taught in order to (i) produce a single, substantially directionally transverse, light beam and (ii) heat substantially omnidirectionally should be understood to be illustrative only, and not delimiting of the potential combinations of heating by irradiating with both light and heat that are subsumed within the present invention.

Therefore, the present invention should be interpreted in accordance with the language of the following claims, only, and not solely in accordance with that particular embodiment within which the invention has been taught.

## Claims

1. A medical device (10) for conducting laser energy from a region outside a patient's body to a region within the body and for applying the laser energy to the body both as radiation and as heat, the device comprising :
(a) an elongated laser energy conduit (22) having a distal end and a proximal end (14) and being placeable from a region outside the body to a region within the body;
(b) a source of laser energy (50) optically coupled to the laser energy conduit (22) for transmitting laser energy from the proximal end region (14) to the distal end region of the conduit;
(c) a beam splitter means (26, 226, 326, 426), located at the distal end region of the elongated laser energy conduit (22) and receiving the laser energy transmitted by the source of laser energy (50) to the distal end region of the conduit (22), for splitting the received laser energy into at least a first portion and a second portion of laser energy;
(d) a hollow bulbous element (18, 218, 318, 418), receiving the first portion of laser energy from the beam splitter means, for converting the received first portion of laser energy into heat, the hollow bulbous element (18, 218, 318, 418) being mounted on the distal end region of the conduit (22) coaxial with the conduit (22), the hollow bulbous element (18, 218, 318, 418) including a cavity (20) therein within which the distal end of the conduit (22) is received; and
(e) an aperture (24, 224, 324, 424), defined by and positioned within the element (18, 218; 318, 418), receiving the second portion of laser energy from the beam splitter means (26, 226, 326, 426) for radiatively communicating the received second portion of laser energy;
characterised in that
the cavity (20) is open to fluid communication from outside the hollow bulbous element (18, 218, 318, 418) through said aperture (24, 224, 324, 424), said aperture (24, 224, 324, 424) being laterally placed for communicating the second portion of laser energy transversely to the axis of the conduit (22) and externally to the medical device (10).

2. A device according to claim 1, wherein the hollow bulbous element (18, 218, 318, 418) includes a light-absorbing substance which converts laser energy impinging upon its surface to heat.

3. A device according to claim 2, wherein the bulbous element's light-absorbing substance is located oppositely the distal end of the conduit (22).

4. A device according to any preceding claim, wherein the size and length of the conduit (22), the size of the hollow bulbous element (18, 218, 318, 418), and the magnitude of the available laser energy coupled to the conduit (22) by the laser energy source (50) are chosen for local application of heat and light energy within the uterus sufficient to destroy the endometrium lining of the uterine wall.

5. A device according to any preceding claim, wherein the hollow bulbous element (18, 218, 318, 418) is constructed so that an outer surface thereof adjacent the aperture (24, 224, 324, 424) is spaced transversely from the distal end of the conduit (22) to define a spacing between the distal end of the conduit and a selected body site when the bulbous element is placed in contact with that body site with the aperture (24, 224, 324, 424) oriented towards the body site.

6. A device according to claim 5, wherein the said spacing is of substantially the same size as the transverse dimension of the conduit (22).

7. A device according to claim 1, wherein the beam splitting means (26, 226, 326, 426) include a partially energy reflective mirror surface (70), receiving the laser energy transmitted by the conduit (22), which communicates a portion of the received laser energy to the hollow bulbous element (18, 218, 318, 418) as the laser energy impinges thereon and which communicates another portion of the received energy to the said element's aperture (24, 224, 324, 424) as the laser energy beam in registry therewith.

8. A device according to claim 7, wherein the partially energy reflective mirror surface is defined by a layer of gold.

9. A device according to claim 7, wherein the partially energy reflective mirror surface communicates the portion of the received energy to the bulbous element by transmission, and the portion of the received energy through the bulbous element's aperture by reflection.

10. A device according to claim 7, wherein the reflectivity of the partially energy reflective mirror surface may be varied in order to adjust the proportion of the received laser energy that is communicated to the bulbous element and that which is communicated to the bulbous element's aperture.

11. A device according to any preceding claim, wherein the element's aperture (24, 224, 324, 424) is located at a position proximate to the distal end of the laser energy conduit (22) and to the beam splitting means (26, 226, 326, 426).

12. A device according to claim 11, wherein the element's aperture (24, 224, 324, 424) is relatively narrower where it is proximate to the laser energy conduit (22) and to the beam splitting means (26, 226, 326, 426) and is relatively wider where it exits the element (18, 218, 318, 418).

13. A device according to any preceding claim, wherein the element's aperture (24, 224, 324, 424) is substantially in the shape of a frustum with the base of the frustum disposed to the exterior of the element (18, 218, 318, 418) and the truncated apex of the frustum abutting the beam splitting means (26, 226, 326, 426).

14. A device according to claim 13, wherein the element's frustum-shaped aperture is substantially frusto-conical.

15. A device according to claim 14, wherein the frusto-conically shaped element's aperture (24, 224, 324, 424) forms an angle of less than 90°.

16. A device according to any preceding claim, wherein the beam splitting means (26, 226, 326, 426) comprise :
(a) a notch within the laser energy conduit at its distal end region for radiatively directing a portion of the laser energy that is transmitted by the source of laser energy (50) to the distal end region of the conduit (22) further to the aperture as the second portion of laser energy; and
(b) a light-receiving surface positioned oppositely to a distal end of the laser energy conduit (22) for receiving another portion of the laser energy transmitted by the source of laser energy to the distal end region of the conduit and for transmitting this portion to the element as the first portion of laser energy.

## Patentansprüche

1. Medizinische Vorrichtung (10) zum Leiten von Laserenergie von einem Bereich außerhalb des Körpers eines Patienten in einen Bereich innerhalb des Körpers und zum Anwenden der Laserenergie auf den Körper sowohl als Strahlung als auch als Wärme, wobei die Vorrichtung aufweist:
(a) einen langgestreckten Laserenergiekanal (22) mit einem körperfernen Ende und einem körpernahen Ende (14), der von einem Bereich außerhalb des Körpers zu einem Bereich innerhalb des Körpers anordbar ist;
(b) eine Laserenergiequelle (50), die optisch an den Laserenergiekanal (22) zum Transmittieren von Laserenergie vom körpernahen Endbereich (14) zum körperfernen Endbereich des Kanals gekoppelt ist;
(c) ein Strahlteilermittel (26, 226, 326, 426), das am körperfernen Endbereich des langgestreckten Laserenergiekanals (22) angeordnet ist und die durch die Laserenergiequelle (50) zum körperfernen Endbereich des Kanals (22) transmittierte Laserenergie aufnimmt, zum Aufteilen der aufgenommenen Laserenergie in mindestens einen ersten Teil und einen zweiten Teil von Laserenergie;
(d) ein kugelförmiges Hohlelement (18, 218, 318, 418), das den ersten Teil von Laserenergie vom Strahlteilermittel aufnimmt, zum Umwandeln des aufgenommenen ersten Teils von Laserenergie in Wärme, wobei das kugelförmige Hohlelement (18, 218, 318, 418) am körperfernen Endbereich des Kanals (22) koaxial mit dem Kanal (22) angebracht ist und darin einen Hohlraum (20) enthält, innerhalb dessen das körperferne Ende des Kanals (22) aufgenommen wird; und
(e) eine durch das Element (18, 218, 318, 418) definierte und innerhalb desselben positionierte Öffnung (24, 224, 324, 424), die den zweiten Teil von Laserenergie vom Strahlteilermittel (26, 226, 326, 426) zur Strahlungsübertragung des aufgenommenen zweiten Teils von Laserenergie aufnimmt;
dadurch gekennzeichnet, daß
der Hohlraum (20) für Flüssigkeitsübertragung von außerhalb des kugelförmigen Hohlelements (18, 218, 318, 418) durch besagte Öffnung (24, 224, 324, 424) offen ist, wobei besagte Öffnung (24, 224, 324, 424) seitlich angeordnet ist, um den zweiten Teil von Laserenergie transversal zur Achse des Kanals (22) und extern zur medizinischen Vorrichtung (10) zu übertragen.

2. Vorrichtung nach Anspruch 1, worin das kugelförmige Hohlelement (18, 218, 318, 418) eine lichtabsorbierende Substanz enthält, die auf ihre Oberfläche auftreffende Laserenergie in Wärme umwandelt.

3. Vorrichtung nach Anspruch 2, worin die lichtabsorbierende Substanz des kugelförmigen Elements dem körperfernen Ende des Kanals (22) gegenüberliegend angeordnet ist.

4. Vorrichtung nach einem der vorangehenden Ansprüche, worin die Größe und Länge des Kanals (22), die Größe des kugelförmigen Hohlelements (18, 218, 318, 418) und die Größenordnung der durch die Laserenergiequelle (50) an den Kanal (22) gekoppelten verfügbaren Laserenergie zur lokalen Anwendung von Wärme und Lichtenergie innerhalb des Uterus hinreichend gewählt werden, um die Endometrium-Auskleidung der Uteruswand zu zerstören.

5. Vorrichtung nach einem der vorangehenden Ansprüche, worin das kugelförmige Hohlelement (18, 218, 318, 418) so konstruiert ist, daß eine an die Öffnung (24, 224, 324, 424) angrenzende äußere Oberfläche hiervon vom körperfernen Ende des Kanals (22) transversal beabstandet ist, um einen Abstand zwischen dem körperfernen Ende des Kanals und einer ausgewählten Körperstelle zu definieren, wenn das kugelförmige Element in Kontakt mit dieser Körperstelle gebracht wird, wobei die Öffnung (24, 224, 324, 424) in Richtung der Körperstelle orientiert ist.

6. Vorrichtung nach Anspruch 5, worin der besagte Abstand von im wesentlichen derselben Größe wie die transversale Ausdehnung des Kanals (22) ist.

7. Vorrichtung nach Anspruch 1, worin das Strahlteilermittel (26, 226, 326, 426) eine die durch den Kanal (22) transmittierte Laserenergie aufnehmende, teilweise energiereflektierende Spiegeloberfläche (70) enthält, die einen Teil der aufgenommenen Laserenergie zum kugelförmigen Hohlelement (18, 218, 318, 418) überträgt, wenn die Laserenergie darauf auftrifft, und die einen weiteren Teil der aufgenommenen Energie zur besagten Öffnung (24, 224, 324, 424) des Elements überträgt, wenn die Laserenergie in Deckung hiermit strahlt.

8. Vorrichtung nach Anspruch 7, worin die teilweise energiereflektierende Spiegeloberfläche durch eine Goldschicht definiert wird.

9. Vorrichtung nach Anspruch 7, worin die teilweise energiereflektierende Spiegeloberfläche den Teil der aufgenommenen Energie zum kugelförmigen Element durch Transmission und den Teil der aufgenommenen Energie durch die Öffnung des kugelförmigen Elements durch Reflexion überträgt.

10. Vorrichtung nach Anspruch 7, worin das Reflexionsvermögen der teilweise energiereflektierenden Spiegeloberfläche variiert werden kann, um das Verhältnis der aufgenommenen Laserenergie, die zum kugelförmigen Element übertragen wird, zu der, die zur Öffnung des kugelförmigen Elements übertragen wird, einzustellen.

11. Vorrichtung nach einem der vorangehenden Ansprüche, worin die Öffnung (24, 224, 324, 424) des Elements an einer an das körperferne Ende des Laserenergiekanals (22) und an das Strahlteilermittel (26, 226, 326, 426) angrenzenden Position angeordnet ist.

12. Vorrichtung nach Anspruch 11, worin die Öffnung (24, 224, 324, 424) des Elements dort im Verhältnis enger ist, wo sie an den Laserenergiekanal (22) und an das Strahlteilermittel (26, 226, 326, 426) angrenzt, und dort im Verhältnis breiter ist, wo sie das Element (18, 218, 318, 418) verläßt.

13. Vorrichtung nach einem der vorangehenden Ansprüche, worin die Öffnung (24, 224, 324, 424) des Elements im wesentlichen in der Form eines Stumpfes ist, wobei die Basis des Stumpfes zum Äußeren des Elements (18, 218, 318, 418) geneigt ist und die abgeschnittene Spitze des Stumpfes am Strahlteilermittel (26, 226, 326, 426) anliegt.

14. Vorrichtung nach Anspruch 13, worin die stumpfförmige Öffnung des Elements im wesentlichen kegelstumpfförmig ist.

15. Vorrichtung nach Anspruch 14, worin die kegelstumpfförmige Öffnung (24, 224, 324, 424) des Elements einen Winkel von weniger als 90° bildet.

16. Vorrichtung nach einem der vorangehenden Ansprüche, worin das Strahlteilermittel (26, 226, 326, 426) aufweist:
(a) eine Aussparung innerhalb des Laserenergiekanals an seinem körperfernen Endbereich zur Strahlungsrichtung eines Teils der Laserenergie, die durch die Laserenergiequelle (50) zum körperfernen Endbereich des Kanals (22) transmittiert wird, weiter zur Öffnung als den zweiten Teil von Laserenergie; und
(b) eine einem körperfernen Ende des Laserenergiekanals (22) gegenüberliegend positionierte, lichtaufnehmende Oberfläche zum Aufnehmen eines weiteren Teils der durch die Laserenergiequelle zum körperfernen Endbereich des Kanals transmittierten Laserenergie und zum Transmittieren dieses Teils zum Element als den ersten Teil von Laserenergie.

## Revendications

1. Dispositif médical (10) pour amener de l'énergie laser depuis une région située à l'extérieur du corps d'un patient jusqu'à une région située à l'intérieur du corps, et pour appliquer l'énergie laser au corps à la fois sous la forme d'un rayonnement et sous celle de chaleur, le dispositif comprenant :
(a) un conduit allongé (22) qui est destiné à l'énergie laser, qui présente une extrémité distale et une extrémité proximale (14) et qui peut être amené depuis une région située à l'extérieur du corps jusqu'à une région située à l'intérieur du corps ;
(b) une source d'énergie laser (50) optiquement couplée au conduit d'énergie laser (22) pour transmettre de l'énergie laser de la région de l'extrémité proximale (14) du conduit à la région de son extrémité distale ;
(c) un moyen de division du rayon (26, 226, 326, 426) qui est situé dans la région de l'extrémité distale du conduit allongé d'énergie laser (22) et qui reçoit l'énergie laser transmise par la source d'énergie laser (50) à la région de l'extrémité distale du conduit (22) pour diviser l'énergie laser reçue en au moins une première partie d'énergie laser et une deuxième partie d'énergie laser ;
(d) un élément bulbeux creux (18, 218, 318, 418) qui reçoit la première partie d'énergie laser depuis le moyen de division du rayon pour transformer en chaleur la première partie d'énergie laser reçue, l'élément bulbeux creux (18, 218, 318, 418) étant monté sur la région de l'extrémité distale du conduit (22) d'une manière coaxiale par rapport au conduit (22), et l'élément bulbeux creux (18, 218, 318, 418) comprenant intérieurement une cavité (20) à l'intérieur de laquelle l'extrémité distale du conduit (22) est reçue ; et :
(e) une ouverture (24, 224, 324, 424) qui est définie par l'élément (18, 218, 318, 418), qui est positionnée à l'intérieur de celui-ci et qui reçoit la deuxième partie d'énergie laser du moyen de division du rayon (26, 226, 326, 426) pour communiquer par rayonnement la deuxième partie d'énergie laser qui est reçue ;
caractérisé par le fait que :
la cavité (20) est ouverte à la communication avec un fluide depuis l'extérieur de l'élément bulbeux creux (18, 218, 318, 418) à travers ladite ouverture (24, 224, 324, 424), ladite ouverture (24, 224, 324, 424) étant placée latéralement pour communiquer la deuxième partie d'énergie laser transversalement par rapport à l'axe du conduit (22) et à l'extérieur du dispositif médical (10).

2. Dispositif selon la revendication 1, dans lequel l'élément bulbeux creux (18, 218, 318, 418) comprend une substance absorbant la lumière pour transformer en chaleur l'énergie laser qui frappe sa surface.

3. Dispositif selon la revendication 2, dans lequel la substance de l'élément bulbeux qui absorbe la lumière est située en face de l'extrémité distale du conduit (22).

4. Dispositif selon l'une quelconque des revendications précédentes, dans lequel la taille et la longueur du conduit (22), la taille de l'élément bulbeux creux (18, 218, 318, 418) et l'intensité de l'énergie laser disponible qui est couplée au conduit (22) par la source d'énergie laser (50) sont choisies en vue d'appliquer localement à l'intérieur de l'utérus de la chaleur et de l'énergie lumineuse en quantité suffisante pour détruire l'endomètre qui tapisse la paroi de l'utérus.

5. Dispositif selon l'une quelconque des revendications précédentes, dans lequel l'élément bulbeux creux (18, 218, 318, 418) est construit d'une manière telle qu'une surface extérieure de celui-ci qui est adjacente à l'ouverture (24, 224, 324, 424) soit espacée dans le sens transversal de l'extrémité distale du conduit (22) pour définir un espacement entre l'extrémité distale du conduit et un site choisi du corps lorsque l'élément bulbeux est mis en contact avec ce site du corps, l'ouverture (24, 224, 324, 424) étant orientée vers le site du corps.

6. Dispositif selon la revendication 5, dans lequel ledit espacement présente sensiblement la même dimension que la dimension transversale du conduit (22).

7. Dispositif selon la revendication 1, dans lequel le moyen de division du rayon (26, 226, 326, 426) comprend une surface d'un miroir qui réfléchit partiellement l'énergie, qui reçoit l'énergie laser transmise par le conduit (22), qui communique une partie de l'énergie laser reçue à l'élément bulbeux creux (18, 218, 318, 418) lorsque l'énergie laser le frappe, et qui communique une autre partie de l'énergie reçue à ladite ouverture (24, 224, 324, 424) de l'élément lorsque le rayon d'énergie laser coïncide avec lui.

8. Dispositif selon la revendication 7, dans lequel la surface du miroir qui réfléchit partiellement l'énergie est définie par une couche d'or.

9. Dispositif selon la revendication 7, dans lequel la surface du miroir qui réfléchit partiellement l'énergie communique par transmission la partie de l'énergie reçue qui est communiquée à l'élément bulbeux et par réflexion la partie de l'énergie reçue qui est communiquée à travers l'ouverture de l'élément bulbeux.

10. Dispositif selon la revendication 7, dans lequel la réflectivité de la surface du miroir qui réfléchit partiellement l'énergie peut être modifiée en vue de régler le rapport entre l'énergie laser reçue qui est communiquée à l'élément bulbeux et celle qui est communiquée à l'ouverture de l'élément bulbeux.

11. Dispositif selon l'une quelconque des revendications précédentes, dans lequel l'ouverture (24, 224, 324, 424) de l'élément est située en un endroit proche de l'extrémité distale du conduit d'énergie laser (22) et proche du moyen de division du rayon (26, 226, 326, 426).

12. Dispositif selon la revendication 11, dans lequel l'ouverture (24, 224, 324, 424) de l'élément est comparativement plus étroite à l'endroit où elle est proche du conduit d'énergie laser (22) et du moyen de division du rayon (26, 226, 326, 426), et qu'elle est comparativement plus large à l'endroit où elle sort de l'élément (18, 218, 318, 418).

13. Dispositif selon l'une quelconque des revendications précédentes, dans lequel l'ouverture (24, 224, 324, 424) de l'élément présente sensiblement la forme d'un volume tronqué, la base du volume tronqué étant disposée à l'extérieur de l'élément (18, 218, 318, 418) et le sommet imaginaire du volume tronqué rencontrant le moyen de division du rayon (26, 226, 326, 426).

14. Dispositif selon la revendication 13, dans lequel l'ouverture en forme de volume tronqué de l'élément est sensiblement tronconique.

15. Dispositif selon la revendication 14, dans lequel l'ouverture (24, 224, 324, 424) de l'élément en forme de tronc de cane forme un angle inférieur à 90°.

16. Dispositif selon l'une quelconque des revendications précédentes, dans lequel le moyen de division du rayon (26, 226, 326, 426) comprend :
(a) une entaille à l'intérieur du conduit d'énergie laser dans la région de son extrémité distale pour diriger par rayonnement une partie de l'énergie laser qui est transmise par la source d'énergie laser (50) à la région de l'extrémité distale du conduit (22), puis à l'ouverture sous la forme de la deuxième partie d'énergie laser ; et :
(b) une surface réceptrice de la lumière qui est placée en face d'une extrémité distale du conduit d'énergie laser (22) pour recevoir une autre partie de l'énergie laser transmise par la source d'énergie laser à la région de l'extrémité distale du conduit et pour transmettre cette partie à l'élément sous la forme de la première partie d'énergie laser.
